(19) Europäisches Patentamt
European Patent Office
Office européen des brevets

(11) **EP 2 052 752 B1**

(12) **EUROPÄISCHE PATENTSCHRIFT**

(45) Veröffentlichungstag und Bekanntmachung des Hinweises auf die Patenterteilung:
**16.11.2011  Patentblatt 2011/46**

(51) Int Cl.:
*A61M 5/168* *(2006.01)*     *A61M 5/142* *(2006.01)*
*A61M 5/145* *(2006.01)*

(21) Anmeldenummer: **07405315.8**

(22) Anmeldetag: **22.10.2007**

(54) **Selbsttätig arbeitende Injektionsvorrichtung und Verfahren zur Ermittlung einer Injektionsokklusion**

Self-functioning injection device and method for detecting an injection occlusion

Dispositif d'injection autonome et procédé de détermination d'une occlusion d'injection

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**29.04.2009  Patentblatt 2009/18**

(73) Patentinhaber:
• **Roche Diagnostics GmbH**
  **68305 Mannheim (DE)**
  Benannte Vertragsstaaten:
  **DE**
• **F. Hoffmann-La Roche AG**
  **4070 Basel (CH)**
  Benannte Vertragsstaaten:
  **AT BE BG CH CY CZ DK EE ES FI FR GB GR HU IE IS IT LI LT LU LV MC MT NL PL PT RO SE SI SK TR**

(72) Erfinder:
• **Brügger, Martin**
  **3098 Köniz (CH)**
• **Oberli, Markus**
  **3422 Kirchberg (CH)**
• **Kühni, Florian**
  **3400 Burgdorf (CH)**
• **Prod'hom, Gilles**
  **2024 St-Aubin (CH)**

(74) Vertreter: **Rüfenacht, Philipp Michael et al**
**Keller & Partner**
**Patentanwälte AG**
**Schmiedenplatz 5**
**Postfach**
**3000 Bern 7 (CH)**

(56) Entgegenhaltungen:
**EP-A- 1 529 546      US-A1- 2004 133 166**
**US-A1- 2007 191 770**

**Beschreibung**

**Technisches Gebiet**

Die Erfindung betrifft eine selbsttätig arbeitende Injektionsvorrichtung mit einer Okklusionsalarmeinheit gemäss dem Oberbegriff des Patentanspruchs 1 sowie ein Verfahren zur Ermittlung einer Injektionsokklusion gemäss dem Oberbegriff des Patentanspruchs 7. Die Injektionseinheit dient zum selbsttätigen Injizieren eines Medikaments in den Körper eines Patienten. In der Regel wird Insulin injiziert, es können aber auch andere, über einen längeren Zeitraum zu injizierende Medikamente, wie z.B. Schmerzmittel injiziert werden.

**Stand der Technik**

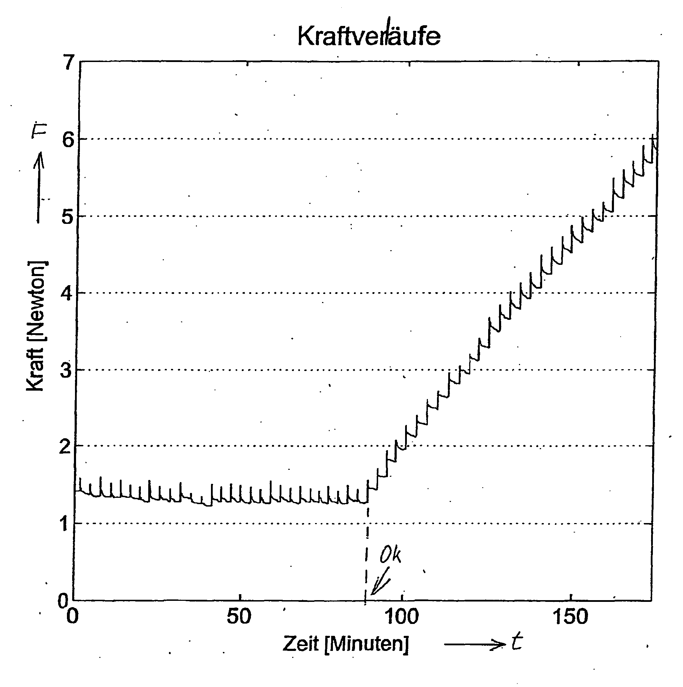

Selbsttätig arbeitende Injektionsvorrichtungen injizieren in den Körper eines Patienten in vorgegebenen Zeitintervallen ein vorgegebenes Volumen eines Medikamentes. Dieses Volumen wird aus einem Speicher (Reservoir), in der Regel einer auswechselbaren Ampulle über einen Pumpenantrieb entnommen und über eine im Körper des Patienten befindliche Injektionsnadel injiziert. Ist nun eine Okklusion vorhanden, so erhöht sich der Druck im injizierenden System, da kein Druckabbau aufgrund von Injektionen erfolgt (siehe **Figur 7**). In **Figur 7** beginnt eine Okklusion zum Zeitpunkt Ok. Eine im Pumpenantrieb aufzuwendende Kraft erhöht sich hierdurch über mehrere, nicht erfolgreich vorgenommene Injektionen. Aus einer Kraftmessung kann somit auf eine vorliegende oder auf keine Okklusion geschlossen werden. Tritt eine Okklusion auf, so wird der Patient einerseits nicht mehr mit einem notwendigen Medikament versorgt; andererseits steigt durch die mit vorgegebenen Zeitintervallen selbsttätig arbeitende Pumpeinheit der Druck in der Ampulle bzw. den Zuleitungen zum Patientenkörper, wodurch die Injektionsvorrichtung Schaden nehmen könnte. Noch bedeutend problematischer ist, dass bei zunehmendem Druck sich plötzlich die Okklusion aufhebt und der Patient dann eine zu grosse Medikamentenmenge erhalten kann. Mit einer Messeinheit, welche eine zum Austreiben des Medikaments erforderliche Kraft ermittelt, kann somit auf eine vorliegende Okklusion geschlossen werden.

In der US 6,659,980 ist beispielsweise eine derartige Injektionseinheit beschrieben, wobei hier zum Austrieb des zu injizierenden Volumens ein mit einer elektromotorisch angetriebenen Spindel vorschiebbarer Kolben verwendet wurde. Um eine Okklusion beim Injizieren festzustellen, schlug die US 6,659,980 ein erstes Verfahren vor, bei dem eine maximale Kraftschwelle vorgegeben war, bei deren Überschreiten ein Okklusionalarm ausgelöst wurde. In einem zweiten Verfahren wurde ein Kraftanstieg mehrerer Kraftwerte für ein Austreiben des Injektionsvolumens aufgenommen. Lag keine Okklusion vor, so war nach den Ausführungen der US 6,659,980 kein zeitlicher Kraftanstieg über mehrere Injektionen ermittelbar; erst beim Auftreten einer Okklusion erfolgte ein Anstieg über mehrere Messungen. Zur Ermittlung des Kraftanstiegs wurden in der US 6,659,980 jeweils in gleichem zeitlichen Intervall ermittelte fünfzehn Kraftwerte ausgewertet.

Es hat sich nun herausgestellt, dass diese bekannte Okklusionsermittlungsmethode langsam war und zu Fehlalarmen führte, welche den Patienten nervte und verunsicherte. In der US 2007/0191770 werden die Merkmale des Oberbegriffs der Ansprüche 1 und 7 offenbart.

**Aufgabe der Erfindung**

Aufgabe der Erfindung ist es, ein Okklusionsermittlungsverfahren und eine selbsttätig arbeitende Injektionsvorrichtung zu schaffen, welche gegenüber dem Stand der Technik eine Okklusion so schnell wie möglich ermitteln und Fehlalarme stark reduzieren kann.

**Lösung der Aufgabe**

Die oben genannte Aufgabe wird dadurch gelöst, dass je nach Auswerteergebnis betreffend einer Okklusion ein Zeitintervall auszuwertender Messwerte, insbesondere von Kraftwerten mittels einer Umstelleinheit nach einer vorgegebenen Anzahl von Injektionen bzw. nach einem vorgegebenen Auswertezeitraum veränderbar, d. h. insbesondere verlängerbar und auch wieder verkürzbar ist. Eine derartige Verlängerung des Messzeitintervalls mit gleichzeitiger Verlängerung des Aufnahme- und Auswertezeitraums, wird vorzugsweise nach acht Stunden bzw. eindeutigem Vorliegen einer Okklusion und nachfolgender Schadensbehebung wieder in den Ausgangszustand zurückgesetzt (verkürzt). Die Veränderung des Messzeitintervalls bzw. des Aufnahme- und Auswertezeitraums erfolgt unabhängig vom Zeitintervall zwischen zeitlich benachbarten Injektionen (Basalausschüttung). In der Regel wird dieses Zeitintervall zwischen aufeinanderfolgenden Injektionen konstant gehalten, könnte aber auch, in Abhängigkeit beispielsweise der Tageszeit, verändert werden. Andere Zeitspannen sind selbstverständlich möglich.

Da vorzugsweise immer eine gleiche Anzahl Messwerte ausgewertet wird, vergrössert sich durch eine Verlängerung des Zeitintervalls auch die Aufnahme- und Auswertezeit der Messwerte. Es hat sich nämlich herausgestellt, dass bei grösseren Beobachtungszeiträumen gegenüber kürzeren eine eindeutigere Aussage über eine Okklusion möglich ist und somit Fehlalarme vermeidbar sind.

Die Messwerte sind mit einer Messeinheit vorzugsweise als direkte bzw. indirekt ermittelte Kraftwerte aufnehmbar. Direkte Kraftwerte wird man mit einem Kraftsensor in Zusammenarbeit mit dem Austriebskolben bzw. dessen Antriebsspindel ermitteln. Bei einer indirekten Kraftmesseinheit kann beispielsweise ein Kraftwert über eine aufgenommene Leistung des Pumpenantriebs ermittelt werden. Auch könnte ein jeweils ausgetriebenes Medikamentenvolumen ermittelt werden. Es könnte jedoch auch eine Druckmessung oder es könnten Antriebsdaten eines Pumpenmotors ausgewertet werden.

Eine Okklusionsbewertung wird nun vorzugsweise mit einer Reihe von Kraftwerten vorgenommen, welche zur Auswertung in einem Speicher abgespeichert werden.

Zur Verbesserung einer Okklusionsauswertung weist nun die Injektionsvorrichtung neben einem ersten Speicher für die gemessenen oder ermittelten Messwerte einen weiteren, zweiten Speicher auf. Im zweiten Speicher sind statistisch aus Experimenten ermittelte Steigungswerte, insbesondere Anstiegswerte von Messwerten in Abhängigkeit von sich vergrössernden Beobachtungszeiträumen abgespeichert. Die abgespeicherten Steigungswerte definieren im zweiten Speicher einen ersten, zweiten und dritten Werteteil. Der erste Werteteil enthält Werte, welche als eine einwandfreie Okklusion interpretierbar sind. Der zweite Werteteil enthält Werte, welche eindeutig keine Okklusion beschreiben und der dritte Werteteil enthält Werte, welche einen dritten Bereich definieren, in dem nicht eindeutig auf eine oder auf keine Okklusion schliessbar ist.

Die im zweiten Speicher abzuspeichernden Werte werden als Diagramm im Labor statistisch aus Experimenten ermittelt, wobei hier im Experiment eine bzw. keine Okklusion eindeutig einstellbar ist (z. B. verschlossene oder offene Injektionsnadel).

Es ist eine Vergleichereinheit vorhanden, mit der im Vergleich mit den im Diagramm abgelegten Daten und mit den Auswertedaten auf eine, keine oder eine nicht bestimmbare Okklusion geschlossen werden kann, je nachdem wo ein aus mehreren Messwerten (Kraftwerten) über einen vorgegebenen Auswertezeitraum ermittelter Anstiegswert (Kraftanstiegswert) im Diagramm zu liegen kommt. Die Vergleichereinheit ist mit der Auswerteeinheit signalmässig verbunden.

Zur konstruktiven Vereinfachung der Injektionsvorrichtung und zur Erleichterung einer Verarbeitung der ermittelten Mess- bzw. dessen Anstiegswerte ist bei einer Vergrösserung des Zeitintervalls aufeinander zu verarbeitender Messwerte bzw. der Aufnahme- und Auswertezeit der Messwerte jeder n-te, im ersten Speicher vorgängig abgespeicherte Messwert mit der Umstelleinheit auswählbar. Es kann nun jeder zweite, dritte, usw. Messwert ausgewählt werden. Vorzugsweise wird man der Einfachheit halber jeden zweiten Messwert auswählen. Die ausgewählten Messwerte (Kraftwerte) sind dann aufeinanderfolgend umspeicherbar und neue, nun mit einem verdoppelten (verdreifachten, usw.) Abtastintervall aufnehmbare Messwerte sind aufeinanderfolgend für einen weiteren, gegenüber dem ersten Beobachtungszeitraum, nun ebenfalls einen analog verdoppelten (verdreifachten, usw.) Zeitraum einspeicherbar. Diese Bearbeitung der Messwerte erlaubt eine einfache Einspeicherprozedur mit einem verkürzten Aufnahmezeitraum, der jedoch einen vergrösserten Beobachtungszeitraum repräsentiert. D. h. für einen Beobachtungszeitraum von einer Stunde bei einer Auswertung jedes zweiten Messwertes, der auf einen vorhängigen, halbstündigen Beobachtungszeitraum folgt, wird nun statt einer Stunde nur noch eine zusätzliche halbe Stunde beobachtet.

Neben dem oben angeführten Auswerten von Messwerten über einem veränderbaren Beobachtungszeitraum kann in einem dritten Speicher ein Okklusionsmessschwellenfixwert abgespeichert werden. Dieser Fixwert ist ein Sicherheitswert, bei dessen Erreichen durch einen Messwert im Auswertezeitraum ein erstes Signal für den Okklusionsalarm auslösbar ist.

Die Injektionsvorrichtung weist eine Messwertsteigungs-, vorzugsweise eine Kraftanstiegsberechnungseinheit auf, welche vorzugsweise als sogenanntes FIR-Filter ("Finite Impulse Response-Filter") ausgebildet ist, und mit der aus den im ersten Speicher abgespeicherten Messwerten eines Auswertezeitraums ein Steigungswert, insbesondere ein Anstiegswert der Messwerte ermittelbar ist, welcher, sofern er von der Auswerteeinheit als im ersten Bereich des Diagramms (= Okklusion) liegend erkennbar ist, ein zweites Signal hervorruft.

Die Injektionsvorrichtung kann neben der oben angeführten Messwertsteigungs- bzw. -anstiegs- bzw. Kraftanstiegsberechnungseinheit eine Medianberechnungseinheit aufweisen. Mit der Medianberechnungseinheit sind mit der von jeweils zwei aufeinander folgenden, im ersten Speicher abspeicherbaren Kraftwerten ermittelte Differenzwerte in aufsteigender Reihe sortierbar. Bei einer ungeraden Anzahl von Messwerten ergibt sich dann eine gerade Anzahl von Differenzwerten. Von den sortierten Differenzwerten ist mit den zwei mittig in der sortierten Differenzwertenreihe liegenden Differenzwerten ein Mittelwert bildbar. Dieser Mittelwert ist mit einem abgespeicherten Okklusionsschwellenkraftwert vergleichbar und bei Erreichen der Schwelle ein drittes Signal erzeugbar. Wurde eine gerade Anzahl von Messwerten verwendet, so hat sich eine ungerade Anzahl von Differenzwerten ergeben. In diesem Fall wird dann der mittig liegende Wert aus den sortierten Differenzwerten verarbeitet.

Für eine Verarbeitung der Messwerte in einem sogenannten FIR-Filter zur Ermittlung eines Steigungs- bzw. eines Anstiegswertes der Messwerte wird man vorzugsweise mit einer ungeraden Anzahl von Messwerten arbeiten, damit, wie untenstehend näher ausgeführt wird, eine Umstellung der Messzeitintervalle sowie der Auswertezeit auf eine einfache Art und Weise vornehmbar ist. Auch für die Ermittlung der Medianberechnung kann diese ungerade Anzahl von Messwerten verwendet werden.

Die oben erhaltenen drei Signale können nun jedes für sich alleine betreffend eines Okklusionsalarmes ausgewertet werden, vorzugsweise wird man jedoch eine Kontrolleinheit mit einer logischen UND-Schaltung und einer nachgeschalteten logischen ODER-Schaltung verwenden. Mit der UND-Schaltung wird das zweite und das dritte Signal verarbeitet und dieses Ausgangsergebnis und das erste Signal mit der ODER-Schaltung verknüpft. Bei einer Erfüllung der ODER-Verknüpfung ist ein Okklusionsalarm auslösbar. Diese Verknüpfung ergibt eine zusätzliche Sicherheit, damit eine vom FIR-Filter eventuell nicht erkannte Okklusion dennoch zu einem Alarm führt. Ferner lässt sich durch diese Anordnung die Möglichkeit eines Fehlalarms betreffend einer vermeintlichen Okklusion signifikant reduzieren.

Aus der nachfolgenden Detailbeschreibung und der Gesamtheit der Patentansprüche ergeben sich weitere vorteilhafte Ausführungsformen und Merkmalskombinationen der Erfindung.

**Kurze Beschreibung der Zeichnungen**

Die zur Erläuterung von Ausführungsbeispielen der Erfindung verwendeten Zeichnungen zeigen in

Fig. 1    einen Grundaufbau einer erfindungsgemässen Injektionseinheit,

Fig. 2    ein Blockschaltbild einer Kontrolleinheit der Injektionseinheit zur Okklusions- detektion, wobei Intervalle zur Auswertung von Messwerten veränderbar sind,

Fig. 3    ein Diagramm, bei dem Kraftanstiegswerte [N/h] (Ordinate) über einem sich ver- grössernden Beobachtungszeitraum [h] (Abszisse) aufgetragen sind,

Fig. 4    eine bildliche Darstellung zur Berechnung des Kraftanstiegs,

Fig. 5    ein Blockschaltbild, in dem eine Okklusionsbewertung durch eine Verknüpfung mit einer Kraftanstiegsermittlung, einer Medianberechnung und einer Fixschwellenbe- wertung erfolgt,

Fig. 6    eine detaillierte Darstellung der in **Figur 5** dargestellten Funktionsblöcke für eine FIR-Filterung und eine Medianberechnung und

Fig. 7    einen Kraftverlauf über die Zeit bei einem Okklusionsbeginn zum Zeitpunkt OK.

**Wege zur Ausführung der Erfindung**

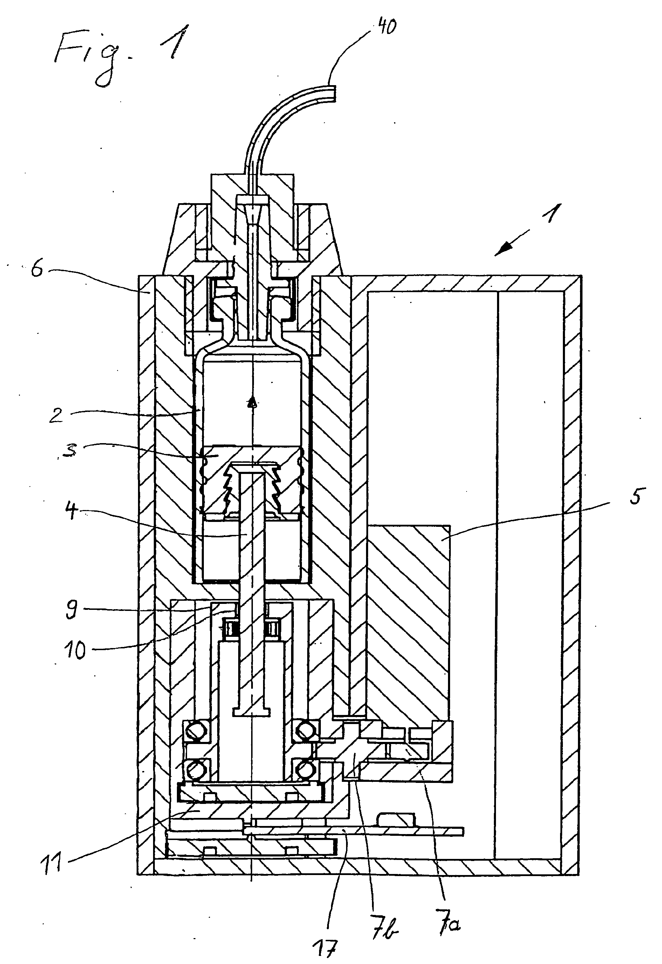

In **Figur 1** ist ein konstruktiver Aufbau einer erfindungsgemässen Injektionsvorrichtung **1** dargestellt, wie sie vorzugsweise zum automatischen Injizieren von Insulin unter die Haut eines Patienten verwendbar ist.

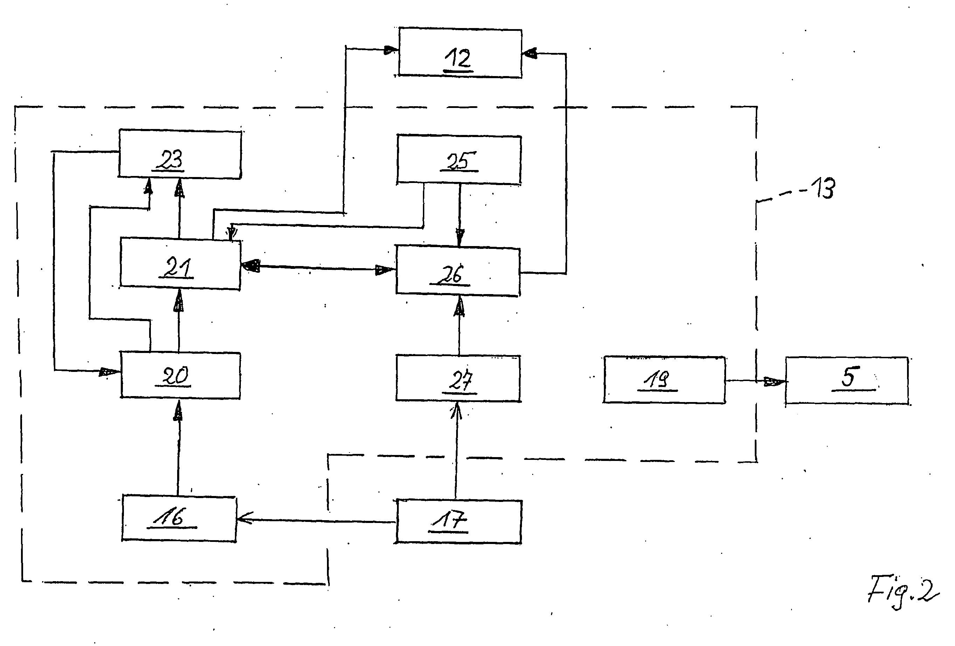

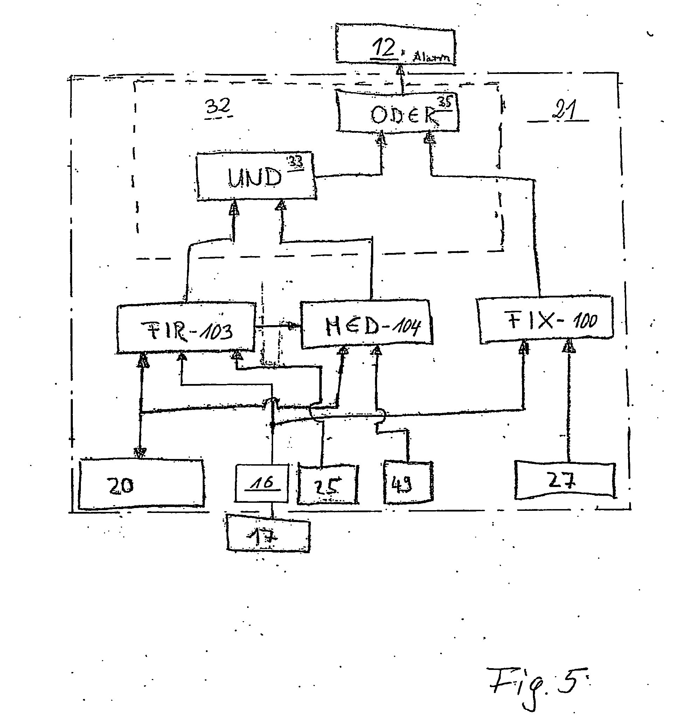

Die Injektionsvorrichtung **1** hat eine in einem Gehäuse **6** untergebrachte Pumpeinrichtung, ein Reservoir **2,** in dem Wirkstoff gespeichert ist, eine nicht dargestellte, auswechselbare Energieversorgungseinheit, eine in den **Figuren 2** und **5** dargestellte Alarmeinheit **12** und eine in **Figur 2** gezeigte Kontrolleinheit **13** u. a. für eine Steuerung der Pumpen- und der Alarmeinheit **12.**

Die Pumpeneinheit hat einen im Reservoir **2** zu liegen kommenden Kolben **3,** der über ein stabförmiges Antriebsglied **4** von einem Elektromotor **5** über Zahnräder **7a** und **7b** angetrieben wird. Der Elektromotor 5 mit den Kraftübertragungselementen - Zahnräder **7a** und **7b,** welche auf ein hülsenartiges weiteres Antriebsglied **9** wirken, das über ein Gewinde **10** mit dem Antriebesglied **4** kämmt - sind auf einer "freischwimmenden" Basis **11** angeordnet, welche auf einen Kraftsensor **17** als Messeinheit zur Ermittlung von für eine Injektion aufzubringenden Kraftwerten F als Messwerte wirkt.

Die in **Figur 2** dargestellte Kontrolleinheit **13** weist neben einer Steuereinheit **19** für den Elektromotor **5** einen ersten Speicher **20** zum Abspeichern von Kraftwerten F auf, welche vom Kraftsensor **17** erfasst und übermittelt werden. Ferner hat die Kontrolleinheit **13** eine Auswerteeinheit **21** zur Ermittlung einer Injektionsokklusion unter einer Verabreitung der im ersten Speicher **20** abgespeicherten Kraftwerte F. Die Kontrolleinheit **13** hat zudem eine mit einem sogenannten Downsampler **16** verbundene Umstelleinheit **23,** mit der in Abhängigkeit eines Auswerteergebnisses der Auswerteeinheit **21** selbsttätig ein Zeitintervall zwischen abzuspeichernden Kraftwerten F und auch selbsttätig ein Aufnahme- und Auswertezeitraum abzuspeichernder, auszuwertender Kraftwerte veränderbar sind. Der Downsampler **16** ist signalmässig zwischen dem Kraftsensor **17** und dem ersten Speicher **20** angeordnet; er ist für die später ausgeführte Intervallveränderung von auszuwertenden Messwerten (Kraftwerte F) zuständig. Die Umstelleinheit **23** ist signalmässig zudem mit dem ersten Speicher **20** und der Auswerteeinheit **21** verbunden.

Die Kontrolleinheit **13** der Injektionsvorrichtung **1** hat ferner einen weiteren, zweiten Speicher **25,** in dem über sich vergrössernde Beobachtungszeiträume mittels Experimenten statistisch ermittelte Kraftanstiegswerte als Steigungswerte in einem Diagramm **37** abgespeichert sind, und eine mit der Auswerteeinheit **21** zusammenarbeitende Vergleichereinheit **26,** mit der im Verleich mit den diagrammartig statistisch aus Experimenten ermittelten, unten beschriebenen Anstiegswerten **37** abgelegten Daten und mit den Auswertedaten auf eine, keine oder auf eine nicht bestimmbare Okklusion schliessbar ist.

Die Kontrolleinheit **13** der Injektionsvorrichtung **1** hat einen dritten Speicher **27,** in dem ein eine Okklusion definierender Okklusionskraftschwellenfixwert abgespeichert ist, bei dessen Überschreiten durch einen Kraftwert F im Auswertezeitraum ein erstes Überschreitungssignal für den Okklusionsalarm auslösbar ist. Der Okklusionskraftschwellenfixwert ist ein Sicherheitswert, mit dem vermieden werden soll, dass kein Druck generiert wird, der eine Ruptur der Infusionsvorrichtung erzeugen kann. Der Okklusionskraftschwellenfixwert ist in dem hier beschriebenen Ausführungsbeispiel auf eine fünfmalige, hintereinander erfolgende Überschreitung von fünfundzwanzig Newton eingestellt.

Bei einer Verwendung von Kraftwerten **F,** wie hier im Ausführungsbeispiel beschrieben, wird auf eine Überschreitung einer Schwelle abgestellt. Statt einer Überschreitung kann jedoch ein Unterschreiten einer Schwelle sich ergeben, sofern statt der Kraftwerte als Messwerte auf einen anderen Messwert, wie beispielsweise ein auszutreibendes Volumen abgestellt wird.

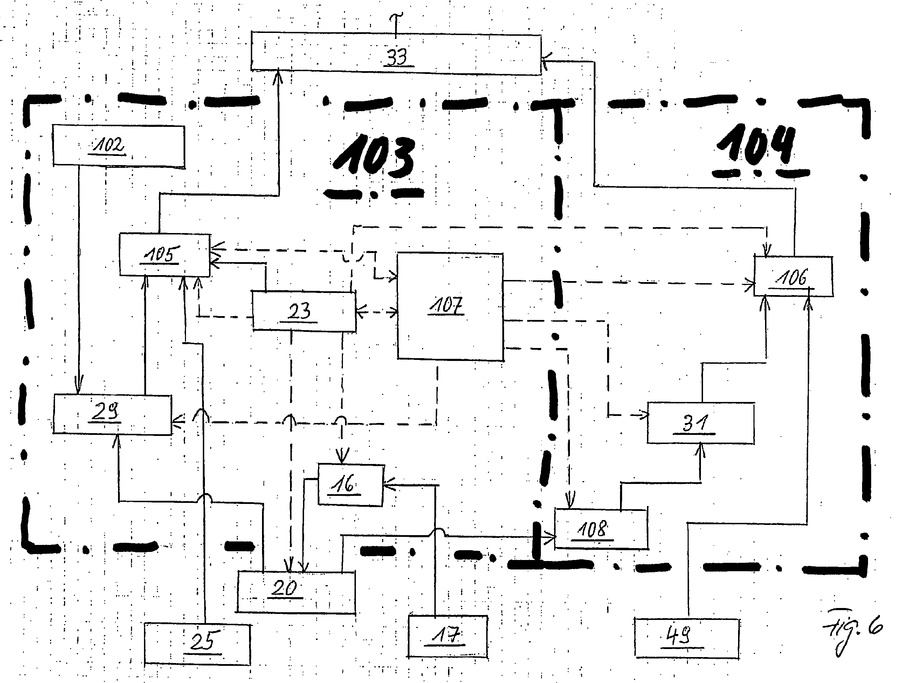

Die Auswerteeinheit **21** der Kontrolleinheit **13** hat eine in **Figur 6** dargestellte Kraftanstiegsberechnungseinheit **29** als Messwertsteigungsberechnungseinheit, welche vorzugsweise als sogenanntes FIR-Filter ausgebildet ist, und mit der aus den in der ersten Speichereinheit **20** abgespeicherten Kraftwerten **F** eines Auswertezeitraums ein Kraftanstiegswert ermittelbar ist, welcher, sofern er von der Auswerteeinheit als im ersten Bereich liegend erkennbar ist, ein zweites Überschreitungssignal hervorruft. Die Funktionsweise der Kraftanstiegsberechnungseinheit **29** ist im Detail unten beschrieben.

Neben der Kraftanstiegsberechnungseinheit **29** hat die Auswerteeinheit **21** vorzugsweise eine Medianberechnungseinheit **31,** mit der von jeweils zwei aufeinander folgenden, im ersten Speicher **20** abspeicherbaren Kraftwerten F ermittelte Differenzwerte **D** in aufsteigender Reihe sortierbar sind. Mit den zwei mittig in der sortierbaren Differenzwertenreihe liegenden Differenzwerten ist ein Mittelwert bildbar. Dieser ist mit einem abgespeicherten Okklusionsschwellenkraftwert vergleichbar, wobei bei Schwellenüberschreitung ein drittes Überschreitungssignal erzeugbar ist.

Die Auswerteeinheit **21** hat ferner insbesondere eine im Detail unten beschriebene Verknüpfungseinheit **32** mit einer logischen UND-Schaltung **33** für das zweite und dritte Überschreitungssignal und mit einer logischen ODER-Schaltung **35** für ein Ausgangssignal der UND-Schaltung **33** und das erste Überschreitungssignal, wobei bei Erfüllung der ODER-Verknüpfung ein Okklusionsalarm mit der Alarmeinheit **12** auslösbar ist.

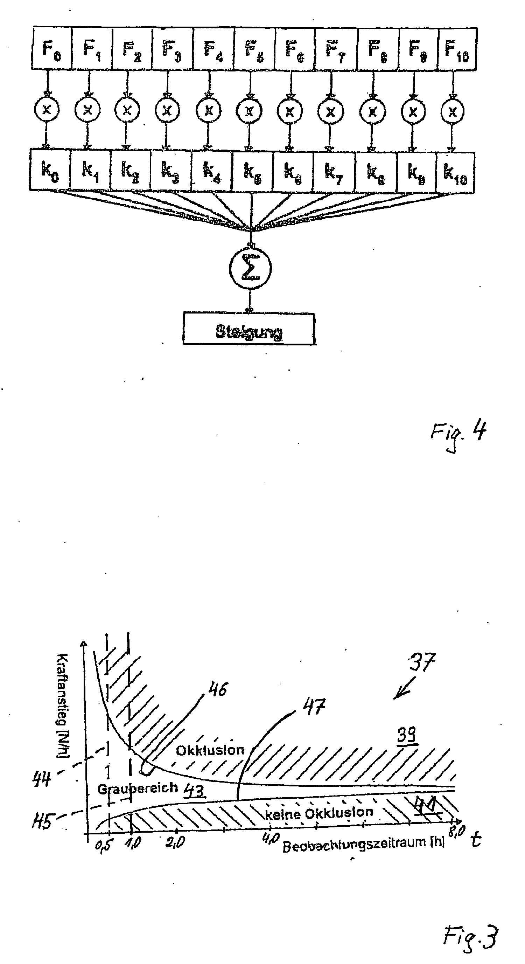

Im zweiten Speicher **25** ist das oben bereits erwähnte, in **Figur 3** dargestellte Diagramm **37** abgespeichert. Das Diagramm **37** wird vorgängig in jede Injektionsvorrichtung **1,** während der Herstellung, d. h. vor der Auslieferung an den Patienten eingespeichert. Das Diagramm **37** enthält Kraftanstiegswerte [N/h], aufgetragen über einem sich vergrössernden Beobachtungszeitraum [h]. Das Diagramm **37** ist vorab aus Experimenten statistisch für jeden Gerätetyp ermittelt worden. Das Diagramm **37** muss nicht für jedes Gerät einzeln erstellt werden; es muss nur einmal pro Gerätetyp experimentell aufgenommen werden. Das Diagramm **37** weist einen ersten Bereich **39** auf, in dem experimentell eindeutig eine Okklusion vorhanden ist. Eine vorhandene Okklusion lässt sich experimentell einwandfrei herstellen, in dem ein Verschluss an einer Injektionsnadel **40** (siehe **Figur 1**) der Injektionsvorrichtung **1** vorgenommen wird. Ein zweiter Bereich **41** des Diagramms **37** definiert eindeutig keine Okklusion. Eine nicht vorhandene Okklusion lässt sich experimentell ebenfalls gut verifizieren, in dem ein Katheter **40,** an dessen äusserem Ende in der Regel eine (nicht dargestellte) Injektionsnadel angeordnet ist, sich ins Freie entleeren kann.

In dem hier angeführten Ausführungsbeispiel werden als Messwerte Kraftwerte ausgewertet, welche zur Injektion eines Medikamentes aufgebracht werden. Einen typischen Kraftverlauf beim Auftreten einer Okklusion zum Zeitpunkt **Ok** zeigt **Figur 7.**

Die für eine Injektion aufzubringende Kraft F ergibt sich aus der nachfolgenden Gleichung zu

$$F = F_0 \, (p) + \varepsilon,$$

wobei $F_0$ eine Funktion des Druckes **p** im zu injizierenden Fluid ist und im Wesentlichen konstant ist. $\varepsilon$ ist eine bei der Injektionsvorrichtung auftretende Störgrösse; es gilt, diese Störgrösse $\varepsilon$ zu eliminieren. $\varepsilon$ hängt von der Reibung des Fluids in den Leitungen, der Kolbenreibung im Reservoir (Ampulle), von der Umgebungstemperatur, von elektrischen Störgrössen, usw. ab.

Ist nun einmal die Injektionsnadel verschlossen und ein anderes Mal frei, so können sich aus den Werten der Kraftanstiegsberechnungseinheit Daten ergeben, welche weder einem Bereich **39** (eindeutige Okklusion) noch einem Bereich **41** (keine Okklusion) zu geordnet werden können. D. h. diese Ergebnisse liegen in einem dritten Bereich **43,** einem Graubereich. In erstaunlicher Weise hat sich nun gezeigt, dass sofern über einen langen Auswertezeitraum gemessen wird, sich dieser Graubereich **43** verkleinert und eine Aussage, ob eine oder keine Okklusion vorliegt, immer besser möglich ist.

Es bieten sich mehrere Methoden an, um eine Steigung bzw. einen Kraftanstieg über einem Auswertezeitraum zu ermitteln. Beispielsweise kann die nachfolgende Methode verwendet werden.

Die Kraftwerte F für jede Injektion werden in Newton kalibriert, wobei generell bei einer Basalausschüttung gemessen wird. Die Messung erfolgt unmittelbar vor dem ersten Burst im jeweiligen Ausschüttungsintervall. Gemessen wird in dem hier aufgezeigten Beispiel alle drei Minuten. Die kalibrierten Kraftwerte F werden im ersten Speicher **20** abgespeichert.

Zur Aufnahme des gerätespezifischen Diagramms werden gemäss einer Theorie zu einer FIR-Filterung in einem ersten Auswertezyklus beispielsweise 11 Kraftwerte $F_0$ bis $F_{10}$ ermittelt und im Speicher **20** abgespeichert. Selbstverständlich kann auch eine gerade Anzahl von Werten verwendet werden. Je weniger Messwerte (Kraftwerte) verwendet werden, desto grösser ist ein Ermittlungsfehler. Viele Werte hingegen verringern diesen Fehler, erhöhen aber die Messzeit. Die im Ausführungsbeispiel vorgeschlagene Anzahl von elf Messwerten hat sich als optimal erwiesen, sowie auch eine zu "0" symmetrische Anordnung der Koeffizienten bei der Berechung der Steigung mittels FIR-Filterung. Eine Theorie zur FIR-Filterung ist beispielsweise in IEEE Transactions on Signal Processing, Vol. 49, No.11, Nov. 2001, S. 2713 - 2730; R. C. Kavanagh, "FIR Differentiators for Quantized Signals" beschrieben.

Diese 11 Kraftwerte $F_0$ bis $F_{10}$ sind bei einem Zeitintervall von drei Minuten zwischen jeweils einer Injektion, also in einem Aufnahmezeitraum von dreissig Minuten, aufgenommen worden.

Zur Ermittlung eines Kraftanstiegs über diesem Aufnahmezeitraum T werden nun die einzelnen, in chronologischer Folge abgelegten Kraftwerte mit jeweils einem Koeffizienten $k_0$ bis $k_{10}$ multipliziert, wobei die Koeffizientenwerte $k_0$ bis $k_{10}$ linear ansteigende Werte zwischen -1 und +1 haben. Die Koeffizienten haben somit die nachfolgenden Werte:

| $k_0$ | $k_1$ | $k_2$ | $k_3$ | $k_4$ | $k_5$ | $k_6$ | $k_7$ | $k_8$ | $k_9$ | $k_{10}$ |
|---|---|---|---|---|---|---|---|---|---|---|
| -1 | -0,8 | -0,6 | -0,4 | -0,2 | 0 | 0,2 | 0,4 | 0,6 | 0,8 | 1,0 |

Der älteste berücksichtigte Wert erhält somit die Gewichtung -1, der neuste Wert wird mit +1 gewichtet. Die derart erhaltenen Multiplikationsergebnisse werden zur Ermittlung des Kraftanstiegs summiert:

$$\text{Kraftanstieg} = \Sigma\,(k_i \times F_i)\,,/\,T \cdot \Sigma\,k_i^2$$

wobei i von 0 bis 10 läuft. Die Ermittlung des Kraftanstiegs zeigt **Figur 4.**

Nach diesem ersten Aufnahmezeitraum erfolgt eine Auswertung gemäss obiger Beschreibung. Anschliessend wird der ermittelte Anstieg mit der unteren und oberen Grenze verglichen. Ausgehend vom Vergleichsresultat ergeben sich drei Möglichkeiten:

    1.) Okklusionsalarm

    2.) Aufnahmezeitraum verdoppeln oder

    3.) Zurück in den Ausgangsmodus (kurzes Messzeitintervall)

Zur Verdopplung des Aufnahmezeitraums wird jeder zweite, im Speicher **20** abgespeicherte Kraftwert **F** eliminiert, dann werden die verbleibenden Kraftwerte **F** aufeinanderfolgend eingespeichert. Im weiteren Aufnahmezeitraum wird mit einem doppelten Zeitintervall gemessen bis eine den vorgängigen Aufnahmezeitraum entsprechende Anzahl Messungen erreicht ist:

| $F_0$ | $F_1$ | $F_2$ | $F_3$ | $F_4$ | $F_5$ | $F6$ | $F_7$ | $F8$ | $F_9$ | $F10$ |
|---|---|---|---|---|---|---|---|---|---|---|

| $F_{n0}= F_0$ | $F_{n1}= F_2$ | $F_{n2}=F_4$ | $F_{n3}=F_6$ | $F_{n4}=F_8$ | $F_{n5}= F_{10}$ | $F_{n6}$ | $F_{n7}$ | $F_{n8}$ | $F_{n9}$ | $F_{n10}$ |
|---|---|---|---|---|---|---|---|---|---|---|

Das tief gestellte "n" soll darauf hindeuten, dass es sich um einen neuen Wertezyklus handelt, der jedoch auch vorhergehende Werte enthält. Die hier dargestellte Umspeicherung lässt sich auch mit einer anderen Anzahl von Kraftwerten vornehmen; eine ungerade Anzahl von Messungen erleichtert aber den Umspeichervorgang.

Gemäss obigem Algorithmus werden sämtliche Erweiterungen des Aufnahmezeitraums beginnend mit einer halben Stunde, dann einer Stunde, dann zwei Stunden, vier Stunden, acht Stunden, ... vorgenommen. Eine Erweiterung auf über acht Stunden ist in der Regel nicht notwendig, da sich bei acht Stunden eines Aufnahmezeitraums der Graubereich bereits sehr stark verringert hat. D. h. nach diesem langen Beobachtungszeitraum ist eine nahezu eindeutige Aussage möglich, ob eine Okklusion oder keine vorgelegen hat.

Das typenspezifisch ermittelte Diagramm **37** wird nun im zweiten Speicher **25** jeder Injektionsvorrichtung **1** abgelegt. Wird die Injektionsvorrichtung **1** vom Patienten oder vom Arzt in Betrieb genommen, so werden bei einer beispielsweisen Ausschüttung eines Medikaments alle drei Minuten, wie oben aufgezeigt, über 30 Minuten die 11 Kraftwerte $F_0$ bis $F_{10}$ gemessen und abgespeichert. Der aus diesen 11 Kraftwerten $F_0$ bis $F_{10}$ ermittelte Kraftanstiegswert wird mit Werten über einem entsprechenden Zeitordninatenwert im abgespeicherten Diagramm **37** verglichen. In **Figur 3** wäre dies für eine halbstündige Beobachtungszeit die Gerade **44** und für eine einstündige Beobachtungszeit die Gerade **45.**

Wird nun zu Beginn einer Medikamentenabgabe mit einem beispielsweisen Zeitintervall zwischen den Basalausschüttungen von drei Minuten nach einem Auswertezeitraum von dreissig Minuten eine Okklusion festgestellt, wobei der Kraftanstiegswert dann im Bereich 39 des Diagramms zu liegen kommt, so wird ein Überschreitungssignal gebildet, welches an die Verknüpfungseinheit weitergegeben wird. Erfolgt nach einem unten beschriebenen Verfahren ein Okklusionsalarm, so werden weitere Ausschüttungen unterbunden. Erfolgt kein Alarm, so wird wie unten stehend beschrieben, verfahren.

Liegt der Kraftanstiegswert im Bereich **43** (Graubereich), in dem auf eine Okklusion nicht eindeutig geschlossen werden kann, so wird, wie oben beschrieben, mit der Umstelleinheit 23 jeder zweite im ersten Speicher **20** abgespeicherte Kraftwert eliminiert, die verbleibenden Kraftwerte aufeinanderfolgend umgespeichert. Neue Kraftwerte werden nun mit einem gegenüber dem ersten Beobachtungszeitraum verdoppelten Abtastintervall aufeinanderfolgend aufgenommen. Der Aufnahmezeitraum ist somit verdoppelt worden.

Kann wieder keine eindeutige Okklusion festgestellt werden (Graubereich), wird diese Prozedur wiederholt.

Wird keine Okklusion festgestellt, so wird keine Verdopplung des Aufnahmezeitraums vorgenommen. Der Wert von $F_{10}$ tritt dann jedoch an die Stelle von $F_{no}$ und die restlichen Werte werden eliminiert. Es wird in den Ausgangsmodus zurückgegangen.

Es sei bemerkt, dass unabhängig von den Zeitintervallen zur Aufnahme der Kraftwerte F das Zeitintervall zwischen den Basalausschüttungen konstant gehalten wird.

Anstatt mit Kraftanstiegswerten zu arbeiten, kann auch mit Kraftwerten gearbeitet werden, wobei sich hier ein anderer Verlauf der Begrenzungslinien zwischen den drei Bereichen ergibt. Die Begrenzungslinien nähern sich hier Geraden an.

Neben einer Auswertung des Kraftanstiegs zur Ermittlung einer Okklusion kann auch eine Medianberechnung bei den ermittelten Kraftwerten innerhalb des Auswertezeitraums vorgenommen werden.

In einem ersten Schritt werden die Differenzen aufeinander folgender Kraftwerte gebildet:

$$D_0 = F_1 - F_2 \quad D_5 = F_6 - F_5$$
$$D_1 = F_2 - F_1 \quad D_6 = F_7 - F_6$$
$$D_2 = F_3 - F_2 \quad D_7 = F_8 - F_7$$
$$D_3 = F_4 - F_3 \quad D_8 = F_9 - F_8$$
$$D_4 = F_5 - F_4 \quad D_9 = F_{10} - F_9$$

Die derart erhaltenen Diffrenzwerte **D** werden nun in aufsteigender Reihenfolge sortiert, aus den Werten von **D4** und **D5** ein Mittelwert gebildet und dieser Mittelwert mit Werten einer Grenzwerttabelle verglichen. Ausgehend vom Diagramm **37** sind für die Medianwerte die nachfolgenden Grenzwerte festgelegt.

| | | | | | |
|---|---|---|---|---|---|
| Grenze zwischen 1. und 3. Bereich **39** und **43; Linie 46** | 8,03 | 7,7 | 7,04 | 5,72 | 4,4 |
| Grenze zwischen 2. und 3. Bereich **41** und **43; Linie 47** | 0,275 | 0,55 | 1,1 | 2,2 | 4,4 |
| Grenzwert "Median" | 0,06 | 0,06 | 0,06 | 0,10 | 0,20 |
| Beobachtungszeit [h] | 0,5 | 1,0 | 2,0 | 4,0 | 8,0 |

Die Grenzwerte betreffend Medianberechnung sind in einem Speicher **49** abgelegt (siehe **Figur 5**). In der Auswertung der Medianwerte wird im Gegensatz zum "Kraftanstieg" eine eindeutige Zuordnung zwischen Okklusion und keiner Okklusion gemacht; einen Graubereich gibt es hier nicht. Auf den Graubereich wurde verzichtet, da wie nachfolgend ausgeführt wird, die Medianwerte zusammen mit den oben ermittelten Kraftanstiegswerten verarbeitet werden. Sollten die Medianwerte alleine, was auch möglich ist, zur Okklusionsbewertung herangezogen werden, kann auch wieder ein Graubereich definiert werden und analog zur Kraftanstiegswertermittlung verfahren werden.

Oben sind drei Verfahren beschrieben, mit denen eine Okklusion erkennbar ist. Jedes dieser Verfahren kann alleine verwendet werden. Es ist jedoch auch möglich, alle drei oder nur jeweils zwei Verfahren miteinander zu verknüpfen. Durch eine Verknüpfung der drei Verfahren ist eine äusserst sichere Feststellung einer Okklusion möglich; Fehlalarme können auf ein Minimum reduziert werden. Bei einer Bolusausschüttung, wie sie bei Insulinpatienten gebräuchlich ist, würde nämlich die Ermittlung über die oben beschriebene Kraftanstiegsberechnung zu einem erhöhten Risiko vor Fehlalarmen führen, sofern keine besondere Softwareroutine für vom Patienten verursachte Bolusausschüttungen zwischengeschaltet würde.

Eine Verknüpfung der drei Verfahren zeigt **Figur 5** in einem Blockschaltbild mit dem Funktionsblock **FIX 100** für eine Verarbeitung des Okklusionskraftschwellenfixwertes, dem Funktionsblock **MED 104** für die Medianberechung und den Funktionsblock **FIR 103** für die FIR-Filterung. Die Signalausgänge der Blöcke **FIR 103** und **Med 104** gehen auf die Eingänge einer UND-Schaltung **33.** Der Ausgang der UND-Schaltung 33 und der Ausgang von **FIX 100** sind zum Eingang einer ODER-Schaltung 35 geführt. Der Ausgang der ODER-Schaltung 35 ist mit der Alarmeinheit **12** verbunden. Ist die ODER-Bedingung erfüllt, wird ein Okklusionsalarm ausgelöst.

**Figur 6** zeigt die beiden Funktionsblöcke **FIR 103** und **Med 104** in einer gegenüber **Figur 5** detaillerteren Darstellung, wobei die ausgezogenen Linien einen Datenfluss und die gestrichelten Linien einen Steuerungsfluss zeigen.

Ein Eingang der UND-Schaltung 33 ist mit einer Vergleichereinheit **105** des Funktionsblockes **FIR 100** verbunden. Der andere Eingang der UND-Schaltung **33** ist mit der Vergleichereinheit **106** verbunden. Eine Kraftanstiegsberechnung der Kraftwerte wird in der Einheit **29** vorgenommen. Eine Medianberechnung wird mit der Einheit **31** vorgenommen. Die Differenzwerte für die Medianberechnung werden im Block **108** vorgenommen. Die für eine Auswertung der Mediane notwendigen Grenzwerte sind im Speicher **49** abgelegt. Eine Steuerung des gesamten Auswerte und Berechnungsablaufes erfolgt mittels des Blockes **107** (Ablaufsteuerung).

Die oben beschriebene Verknüpfung der drei Auswerteverfahren "Fixwert", "FIR-Filterung" und "Medianberechnung" kann auch ohne eine zwingende Aufnahmezeitveränderung vorgenommen werden.

Wurde ein Okklusionsalarm ausgelöst, so kann vorzugsweise eine Wiedereinschaltung nur vorgenommen werden, wenn die Okklusion auch sicher behoben worden ist. Hierzu wird eine erste Basalausschüttung initiiert. Ist der hierbei gemessene Kraftwert grösser als 90% des vorhergehenden Kraftwertes, der zur Alarmauslösung führte, so bleibt der Alarm weiterhin bestehen und es kann keine weitere Basalausschüttung initiiert werden. Vorzugsweise wird man die Ermittlung des augenblicklichen Kraftwertes im Verhältnis zum vorhergehenden Kraftwert mit einem Zeitintervall verknüpfen. Der Prozentwert des Kraftwertes und das zugelassene Zeitintervall hängen von den jeweiligen Daten der Injektionsvorrichtung ab.

Oben stehend sind bei einer Okklusion ansteigende Werte ausgewertet worden. Bei einer Okklusion können sich jedoch bei anderen Messverfahren auch abfallende Messwerte ergeben. Ein derartiges Messverfahren ist z. B. in der WO 2007/093064 A1 beschrieben. Abfallende Messwerte können analog zu den oben beschriebenen ansteigenden verarbeitet werden.

**Patentansprüche**

**1.** Selbsttätig arbeitende Injektionsvorrichtung (1) mit einer Messeinheit (17) zur Ermittlung von mit vorzunehmenden Injektionen eines Medikaments in Zusammenhang stehenden Messwerten (F), insbesondere zur Ermittlung von jeweils einem Kraftwert (F) mehrerer in einem zeitlichen Abstand voneinander vornehmbaren Injektionen, einem Speicher (20) zum Abspeichern der Messwerte (F), einer Auswerteeinheit (21) zur Ermittlung einer Injektionsokklusion aus den abgespeicherten Messwerten (F) und einer Okklusionsalarmeinheit (12), wobei eine Umstelleinheit (23), mit der in Abhängigkeit eines Auswerteergebnisses der Auswerteeinheit (21) und unabhängig vom Zeitintervall zwischen zeitlich benachbarten Injektionen selbsttätig ein Zeitintervall zwischen abzuspeichernden Messwerten (F) veränderbar ist, wodurch sich selbsttätig ein Aufnahme- und Auswertezeitraum abzuspeichernder, auszuwertender Messwerte (F) verändert, **gekennzeichnet durch** einen weiteren, zweiten Speicher (25), in dem statistisch aus Experimenten ermittelte Messwerte bzw. Steigungswerte, insbesondere Anstiegswerte, der Messwerte in Abhängigkeit von sich vergrössernden Beobachtungszeiträumen, einen ersten, zweiten und dritten Werteteil bildend, abgespeichert sind, wobei der erste Werteteil der abgespeicherten Werte (37) einen ersten Bereich (39) definiert, in dem eine Okklusion vorhanden ist, der zweite Werteteil einen zweiten Bereich (41) definiert, in dem keine Okklusion vorhanden ist, sowie der dritte Werteteil einen zwischen dem ersten und dem zweiten Bereich (39, 41) liegenden, dritten Bereich (43) definiert, in dem nicht eindeutig auf eine Okklusion schliessbar ist und eine mit der Auswerteeinheit (21) zusammenarbeitenden Vergleichereinheit (26), mit der im Vergleich mit den im zweiten Speicher (25) abgespeicherten Daten (37) und mit den Auswertedaten auf eine, keine oder auf eine nicht bestimmbare Okklusion schliessbar ist.

2.  Injektionsvorrichtung (1) nach Anspruch 1, **dadurch gekennzeichnet, dass** mit der Umstelleinheit (23) jeder n-te, vorzugsweise jeder zweite, im ersten Speicher (20) abgespeicherte Messwert (F) auswählbar ist, die ausgewählten Messwerte (F) aufeinanderfolgend umspeicherbar sind und neue, nun mit einem entsprechend vergrösserten, vorzugsweise verdoppelten Abtastintervall aufnehmbare Messwerte (F) aufeinanderfolgend für einen weiteren, gegenüber dem ersten Beobachtungszeitraum, nun ebenfalls einen analog vergrösserten Zeitraum aufweisend, einspeicherbar sind.

3.  Injektionsvorrichtung (1) nach einem der Ansprüche 1 oder 2, **gekennzeichnet durch** einen dritten Speicher (27), in dem ein eine Okklusion definierender Okklusionskraftschwellenfixwert abgespeichert ist, bei dessen Erreichen **durch** einen Messwert (F) im Auswertezeitraum ein erstes Signal für den Okklusionsalarm auslösbar ist.

4.  Injektionsvorrichtung (1) nach einem der Ansprüche 1 bis 3, **gekennzeichnet durch** eine Messwertsteigungsberechnungseinheit (FIR 103), welche vorzugsweise als sogenanntes FIR-Filter ("Finite-Impulse-Response-Filter") ausgebildet ist, und mit der aus den im ersten Speicher (20) abgespeicherten Messwerten (F) eines Auswertezeitraums ein Steigungswert, insbesondere ein Anstiegswert der Messwerte ermittelbar ist, welcher, sofern er von der Auswerteeinheit (21) als im ersten Bereich liegend erkennbar ist, ein zweites Signal hervorruft.

5.  Injektionsvorrichtung (1) nach einem der Ansprüche 1 bis 4, **gekennzeichnet durch** eine Medianberechnungseinheit (MED 104), mit der aus einer vorgebbaren Anzahl von Differenzwerten aufeinanderfolgender Messwerte (F) ein Medianwert ermittelbar ist, dieser Medianwert mit einem abgespeicherten Okklusionsschwellenmess-, insbesondere -kraftwert vergleichbar und bei Erreichen der Schwelle ein drittes Signal erzeugbar ist.

6.  Injektionsvorrichtung (1) nach Anspruch 5, wenn abhängig von den Ansprüchen 3 und 4, **gekenntzeichnet durch** eine Verknüpfungseinheit (32) mit einer logischen UND-Schaltung (33) für das zweite und dritte Signal und mit einer logischen ODER-Schaltung (35) für einen Ausgangswert der UND-Schaltung (33) und das erste Signal, wobei bei Erfüllung der ODER-Verknüpfung ein Okklusionsalarm auslösbar ist.

7.  Verfahren zur Ermittlung einer Injektionsokklusion aus Messwerten (F), insbesondere Injektionskraftwerten (F), beim automatischen Injizieren eines Medikamentes mit einer selbsttätig arbeitenden Injektionsvorrichtung (1) und einem Vergleich der Messwerte (F) mit abgespeicherten Werten sowie einem Auslösen eines Alarms bei einer Okklusion, wobei in Abhängigkeit eines aus den Messwerten (F) zu ermittelnden Injektionsokklusionsauswerteergebnisses und unabhängig vom Zeitintervall zwischen zeitlich benachbarten Injektionen ein Zeitintervall zwischen den abzuspeichernden Messwerten (F) verändert wird, wodurch ein Aufnahme- und Auswertezeitraum für die abzuspeichernden bzw. abgespeicherten Messwerte (F) sich verändern, **dadurch gekennzeichnet, dass** vorgängig in jede Injektionsvorrichtung (1) in Experimenten statistisch ermittelte Messwerte bzw. Steigungswerte, insbesondere Anstiegswerte von Messwerten in Abhängigkeit von sich vergrössernden Beobachtungszeiträumen einen ersten, zweiten und dritten Werteteil bildend abgespeichert werden, wobei der erste Werteteil der abgespeicherten Werte (37) einen ersten Bereich (39) definiert, in dem eine Okklusion vorhanden ist, der zweite Werteteil einen zweiten Bereich (41) definiert, in dem keine Okklusion vorhanden ist, sowie der dritte Werteteil einen zwischen dem ersten und dem zweiten Bereich (39, 41) liegenden dritten Bereich (43) definiert, in dem nicht eindeutig auf eine Okklusion schliessbar ist und die im Aufnahmezeitraum ermittelten Messwerte, insbesondere Kraftwerte (F) ausgewertet und mit den abgespeicherte Daten (37) zur Okklusionsalarmermittlung verglichen werden.

8. Verfahren nach Anspruch 7, **dadurch gekennzeichnet, dass** ein vorgegebener Okklusionsmessschwellenfixwert abgespeichert ist, mit dem die gemessenen Mess- bzw. Kraftwerte (F) verglichen werden und bei Erreichen der Schwelle ein erstes Signal für einen Okklusionsalarm erzeugt wird.

9. Verfahren nach einem der Ansprüche 7 oder 8, **dadurch gekennzeichnet, dass** nach einem Auswertezeitraum, in dem eine Okklusion nicht eindeutig ermittelt wurde, ein Aufnahmezeitraum folgt, in dem jeder n-te, vorzugsweise jeder zweite, eingespeicherte Messwert (F) ausgewählt wird, die ausgewählten Messwerte (F) aufeinanderfolgend eingespeichert werden und im weiteren Aufnahmezeitraum mit einem vergrösserten, vorzugsweise doppelten Zeitintervall gemessen wird, bis eine dem vorgängigen Aufnahmezeitraum entsprechende Anzahl Messungen erreicht ist.

10. Verfahren nach einem der Ansprüche 7 bis 9, **dadurch gekennzeichnet, dass** eine ungerade Anzahl abzuspeichernder Messwerte in jeweils einem Auswertezeitraum gewählt wird.

11. Verfahren nach einem der Ansprüche 7 bis 10, **dadurch gekennzeichnet, dass** zur Ermittlung von Steigungs-, insbesondere Anstiegswerten der Messwerte, vorzugsweise analog einer FIR-Filterung, die in einer zeitlichen Folge abgespeicherten Messwerte (F) mit Koeffizienten, welche zwischen einem oberen und einem unteren Wert, vorzugsweise linear zwischen -1 und +1 liegen, multipliziert, dann addiert und anschliessend der Wert mit einem entsprechenden Wert der eingespeicherten Werte des ersten bis dritten Bereichs (39, 41, 43) verglichen und sofern der Steigungs- bzw. Anstiegswert im zweiten Bereich (41) liegt, in eine Grundeinstellung, vorzugsweise unter einer Verkleinerung des Messzeitintervalls, zurückgegangen wird.

12. Verfahren nach einem der Ansprüche 7 bis 11, **dadurch gekennzeichnet, dass** eine Medianermittlung von Differenzwerten (D) jeweils zweier aufeinander folgender Messwerte (F) vorgenommen wird, dieser Medianwert mit einem abgespeicherten Okklusionsschwellenmess-, insbesondere -kraftwert verglichen wird und bei Erreichen der Schwelle ein drittes Signal erzeugt wird.

13. Verfahren nach Anspruch 12, wenn abhängig von den Ansprüchen 8 und 11, **dadurch gekennzeichnet, dass** das zweite und dritte Signal mittels einer logischen UND-Verknüpfung (33) und das Ausgangsergebnis der UND-Verknüpfung (33) zusammen mit dem ersten Signal in einer logischen ODER-Verknüpfung (35) verarbeitet werden und bei Erfüllung der ODER-Verknüpfung (35) ein Okklusionsalarm ausgelöst wird.

**Claims**

1. Automatically operating injection device (1) with a measurement unit (17), for determination of measurement values (F) associated with injections of a medicament that are to be performed, in particular for determination of in each case a force value (F) of a plurality of injections to be performed at a time interval from one another, a memory (20) for storing the measurement values (F), an evaluation unit (21) for determining an injection occlusion from the stored measurement values (F) and an occlusion alarm unit (12), wherein a switching unit (23) with which a time interval between measurement values (F) to be stored can be automatically modified as a function of an evaluation result from the evaluation unit (21) and independently of the time interval between successive injections, as a result of which a recording and evaluation period of measurement values (F) to be stored and to be evaluated is automatically modified, **characterized by** a further, second memory (25) in which measurement values determined statistically from experiments, and gradient values, in particular increase values, of the measurement values are stored as a function of increasing observation periods, forming a first, second and third value part, where the first value part of the stored values (37) defines a first area (39) in which an occlusion is present, the second value part defines a second area (41) in which no occlusion is present, and the third value part defines a third area (43) which lies between the first and second areas (39, 41) and in which it is not possible to conclude unambiguously that there is an occlusion, and a comparator unit (26) which interacts with the evaluation unit (21) and with which, by comparison with the data (37) stored in the second memory (25) and with the evaluation data, it is possible to conclude that there is an occlusion, that there is no occlusion or that there is no determinable occlusion.

2. Injection device (1) according to Claim 1, **characterized in that** every nth, preferably every second, measurement value (F) stored in the first memory (20) can be selected with the switching unit (23), the selected measurement values (F) can be relocated in succession, and new measurement values (F), now recordable with a correspondingly extended, preferably doubled scanning interval, can be stored in succession for a further period, now likewise analogously extended compared to the first observation period.

3. Injection device **(1)** according to one of Claims 1 or 2, **characterized by** a third memory **(27)** for storing an occlusion force threshold fixed value which defines an occlusion and which, when reached by a measurement value **(F)** in the evaluation period, causes a first signal to be triggered for the occlusion alarm.

4. Injection device **(1)** according to one of Claims 1 to 3, **characterized by** a measurement value increase calculation unit **(FIR 103)** which is preferably designed as a so-called FIR filter (Finite-2mpulse-Response filter) and with which, from the measurement values **(F)** of an evaluation period that are stored in the first memory **(20),** it is possible to determine a gradient value, in particular an increase value, of the measurement values, which, if it is detectable by the evaluation unit **(21)** as lying in the first area, causes a second signal.

5. Injection device **(1)** according to one of Claims 1 to 4, **characterized by** a median calculation unit **(MED 104)** with which a median value can be determined from a predefinable number of differential values of successive measurement values **(F),** this median value can be compared with a stored occlusion threshold measurement value, in particular force value, and a third signal can be generated when the threshold is reached.

6. Injection device **(1)** according to Claim 5, where dependent on Claims 3 and 4, **characterized by** a combinational logic unit **(32)** with a logic AND circuit **(33)** for the second and third signals and with a logic OR circuit **(35)** for an output value of the AND circuit **(33)** and the first signal, and an occlusion alarm can be triggered if the OR logic is true.

7. Method for determining an injection occlusion from measurement values **(F),** in particular injection force values **(F),** in automatic injection of a medicament with an automatically operating injection device **(1)** and a comparison of the measurement values **(F)** with stored values and a triggering of an alarm in the event of an occlusion, wherein, as a function of an injection occlusion evaluation result to be determined from the measurement values **(F)** and independently of the time interval between successive injections, a time interval between the measurement values **(F)** to be stored is modified, as a result of which a recording period and evaluation period for the measurement values **(F)** to be stored are modified, **characterized in that**, in each injection device **(1),** measurement values determined statistically in experiments, or gradient values, in particular increase values, of measurement values, are stored which, as a function of increasing observation periods, form a first, second and third value part, where the first value part of the stored values **(37)** defines a first area **(39)** in which an occlusion is present, the second value part defines a second area **(41)** in which no occlusion is present, and the third value part defines a third area **(43)** which lies between the first and second areas **(39, 41)** and in which it is not possible to conclude unambiguously that there is an occlusion, and the measurement values determined in the recording period, in particular force values **(F),** are evaluated and compared with the stored data **(37)** for occlusion alarm determination.

8. Method according to Claim 7, **characterized in that** a predefined occlusion measurement threshold fixed value is stored with which the measured measurement values or force values **(F)** are compared and, if the threshold is reached, a first signal for an occlusion alarm is generated.

9. Method according to one of Claims 7 or 8, **characterized in that**, after an evaluation period in which an occlusion has not been unambiguously determined, a recording period follows in which every nth, preferably every second, stored measurement value **(F)** is selected, the selected measurement values **(F)** are stored in succession and, in the further recording period, measurement is carried out with an extended, preferably doubled time interval until a number of measurements corresponding to the preceding recording period is reached.

10. Method according to one of Claims 7 to 9, **characterized in that** an uneven number of measurement values to be stored is chosen in each evaluation period.

11. Method according to one of Claims 7 to 10, **characterized in that**, in order to determine gradient values, in particular increase values of the measurement values, preferably analogously to FIR filtering, the measurement values **(F)** stored in a time sequence are multiplied by coefficients lying between an upper value and lower value, preferably linearly between -1 and +1, then added, after which the value is compared with a corresponding value of the stored values of the first to third areas **(39, 41, 43)** and, if the gradient value or increase value lies in the second area **(41),** is returned to a basic setting, preferably with reduction of the measurement time interval.

12. Method according to one of Claims 7 to 11, **characterized in that** a median of differential values **(D)** of in each case two successive measurement values **(F)** is determined, this median value is compared with a stored occlusion threshold measurement value, in particular force value, and a third signal is generated when the threshold is reached.

**13.** Method according to Claim 12, where dependent on Claims 8 and 11, **characterized in that** the second signal and third signal are processed by means of a logic AND gate **(33),** and the output result of the AND gate **(33)** is processed together with the first signal in a logic OR gate **(35),** and an occlusion alarm is triggered if the OR gate **(35)** is true.

**Revendications**

**1.** Dispositif d'injection à fonctionnement automatique (1) comprenant une unité de mesure (17) destinée à déterminer des valeurs de mesure (F) en rapport avec des injections de médicament à effectuer, notamment destinée à déterminer une valeur de force (F) respective de multiples injections pouvant être effectuées de manière qu'elles soient espacées les unes des autres dans un intervalle de temps, une mémoire (20) destinée à mémoriser les valeurs de mesure (F), une unité d'évaluation (21) destinée à déterminer une occlusion d'injection à partir des valeurs de mesure (F) mémorisées et une unité d'alarme d'occlusion (12), comprenant une unité de sollicitation (23) permettant de modifier automatiquement un intervalle de temps entre des valeurs de mesure (F) à mémoriser d'une manière qui dépend d'un résultat d'évaluation de l'unité d'évaluation (21) et d'une manière qui ne dépend pas de l'intervalle de temps entre des injections voisines dans le temps, cela ayant pour conséquence de modifier automatiquement une période d'acquisition et une période d'évaluation des valeurs de mesure (F) à mémoriser, **caractérisé par** une deuxième mémoire (25) supplémentaire dans laquelle sont mémorisées des valeurs de mesure ou des valeurs de pente, notamment des valeurs d'accroissement des valeurs de mesure, déterminées statistiquement à partir d'expériences, d'une manière qui dépend de périodes d'observation croissantes en formant des première, deuxième et troisième valeurs partielles, dans lequel la première valeur partielle des valeurs mémorisées (37) définit un premier domaine (39) dans lequel il se produit une occlusion, la deuxième valeur partielle définit un deuxième domaine (41) dans lequel il ne se produit aucune occlusion et la troisième valeur partielle définit un troisième domaine (43) se situant entre les premier et deuxième domaines (39, 41) dans lequel il est impossible de conclure formellement à une occlusion, et une unité de comparaison (26) coopérant avec l'unité d'évaluation (21), au moyen de laquelle il est possible de conclure à une occlusion, à l'absence d'occlusion ou à une occlusion ne pouvant pas être déterminée par comparaison aux données (37) mémorisées dans la deuxième mémoire (25) et aux données d'évaluation.

**2.** Dispositif d'injection (1) selon la revendication 1, **caractérisé en ce que** l'unité de sollicitation (23) permet de sélectionner chaque n-ème, et de préférence, chaque deuxième valeur de mesure (F) mémorisée dans la première mémoire (20), **en ce que** les valeurs de mesure (F) sélectionnées peuvent être restaurées séquentiellement et **en ce que** de nouvelles valeurs de mesure (F) pouvant maintenant être acquises avec un intervalle d'échantillonnage accru, de préférence doublé, peuvent être mémorisées séquentiellement pour une autre période de temps, elle aussi augmentée de manière semblable, par rapport à la première période d'observation.

**3.** Dispositif d'injection (1) selon l'une des revendications 1 ou 2, **caractérisé par** une troisième mémoire (27) dans laquelle est mémorisée une valeur fixe de seuil de force d'occlusion définissant une occlusion, un premier signal étant déclenché aux fins de l'alarme d'occlusion lorsque ce seuil est atteint par une valeur de mesure (F) pendant la période d'évaluation.

**4.** Dispositif d'injection (1) selon l'une des revendications 1 à 3, **caractérisé par** une unité de calcul de pente des valeurs de mesure (FIR 103) qui est de préférence réalisée sous la forme d'un filtre dit FIR ("Finite-Impulse-Response") et au moyen de laquelle une valeur de pente, notamment une valeur d'accroissement des valeurs de mesure peut être déterminée à partir des valeurs de mesure (F) d'une période d'évaluation mémorisées dans la première mémoire (20), laquelle valeur de pente déclenche un deuxième signal si l'unité d'évaluation (21) détecte qu'elle se situe dans le premier domaine.

**5.** Dispositif d'injection (1) selon l'une des revendications 1 à 4, **caractérisé par** une unité de calcul de médiane (MED 104) au moyen de laquelle une valeur de médiane peut être déterminée à partir d'un nombre pouvant être prédéterminé de valeurs de différence entre des valeurs de mesure (F) consécutives, en ce que ladite valeur de médiane peut être comparée à une valeur de mesure de seuil d'occlusion, notamment à une valeur de force, et en ce qu'un troisième signal peut être généré lorsque le seuil est atteint.

**6.** Dispositif d'injection (1) selon la revendication 5 lorsqu'elle dépend des revendications 3 et 4, **caractérisé par** une unité de combinaison (32) comprenant une porte logique ET (33) destinée aux deuxième et troisième signaux et une porte logique OU (35) destinée à une valeur de sortie de la porte ET (33) et au premier signal, dans lequel une alarme d'occlusion peut être déclenchée lorsque la combinaison OU est satisfaite.

7. Procédé destiné à déterminer une occlusion d'injection à partir de valeurs de mesure (F), notamment de valeurs de force d'injection (F) lors de l'injection automatique d'un médicament à l'aide d'un dispositif d'injection à fonctionnement automatique (1) et par une comparaison des valeurs de mesure (F) à des valeurs mémorisées ainsi que par un déclenchement d'une alarme lors d'une occlusion, dans lequel un intervalle de temps entre les valeurs de mesure (F) à mémoriser est modifié d'une manière qui dépend d'un résultat d'évaluation d'occlusion d'injection à déterminer à partir des valeurs de mesure (F) et d'une manière qui ne dépend pas de l'intervalle de temps entre des injections voisines dans le temps, cela ayant pour conséquence de modifier une période d'acquisition et une période d'évaluation des valeurs de mesure (F) à mémoriser, **caractérisé en ce que** des valeurs de mesure ou des valeurs de pente, notamment des valeurs d'accroissement de valeurs de mesures déterminées statistiquement lors d'expériences, sont préalablement mémorisées dans chaque dispositif d'injection (1) d'une manière qui dépend de périodes d'observation croissantes en formant des première, deuxième et troisième valeurs partielles, dans lequel la première valeur partielle des valeurs mémorisées (37) définit un premier domaine (39) dans lequel il se produit une occlusion, la deuxième valeur partielle définit un deuxième domaine (41) dans lequel il ne se produit aucune occlusion et la troisième valeur partielle définit un troisième domaine (43) se situant entre les premier et deuxième domaines (39, 41) dans lequel il est impossible de conclure formellement à une occlusion, et dans lequel les valeurs de mesure déterminées pendant la période d'acquisition, notamment les valeurs de force (F) sont évaluées et sont comparées aux données (37) mémorisées pour la détermination d'une alarme d'occlusion.

8. Procédé selon la revendication 7, **caractérisé en ce qu'**une valeur fixe de seuil d'occlusion prédéterminée est mémorisée et à laquelle les valeurs de mesure ou de force (F) mesurées sont comparées, et **en ce qu'**un premier signal destiné à une alarme d'occlusion est généré lorsque le seuil est atteint.

9. Procédé selon l'une des revendications 7 ou 8, **caractérisé en ce qu'**après une période d'évaluation lors de laquelle une occlusion n'a pas été déterminée formellement, suit une période d'acquisition lors de laquelle chaque n-ême, et de préférence, chaque deuxième valeur de mesure (F) mémorisée est sélectionnée, **en ce que** les valeurs de mesure (F) sélectionnées sont mémorisées séquentiellement et **en ce qu'**elles sont mesurées lors de l'autre période d'acquisition avec un intervalle de temps accru, de préférence doublé, jusqu'à ce qu'un nombre de mesures correspondant à la période d'acquisition préalable soit atteint.

10. Procédé selon l'une des revendications 7 à 9, **caractérisé en ce qu'**un nombre impair de valeurs de mesure à mémoriser est sélectionné pendant une période d'évaluation respective.

11. Procédé selon l'une des revendications 7 à 10, **caractérisé en ce que**, pour la détermination de valeurs de pente, notamment d'accroissement des valeurs de mesure, de préférence de manière semblable à un filtrage FIR, les valeurs de mesure (F) mémorisées selon une séquence temporelle sont multipliées par des coefficients qui se situent entre des valeurs supérieure et inférieure, et varient de préférence linéairement entre -1 et +1, puis sont additionnées et enfin, la valeur est comparée à une valeur correspondante des valeurs mémorisées des premier à troisième domaines (39, 41, 43), et est renvoyée si la valeur de pente ou d'accroissement se situe dans le deuxième domaine (41), dans une configuration de base, de préférence avec diminution de l'intervalle de temps de mesure.

12. Procédé selon l'une des revendications 7 à 11, **caractérisé en ce qu'**on effectue une détermination de la médiane de valeurs de différence (D) entre deux valeurs de mesure (F) consécutives respectives, **en ce que** ladite valeur de médiane est comparée à une valeur de mesure de seuil d'occlusion mémorisée, notamment une valeur de force, et **en ce qu'**un troisième signal est généré lorsque le seuil est atteint.

13. Procédé selon la revendication 12 lorsqu'elle dépend des revendications 8 et 11, **caractérisé en ce que** les deuxième et troisième signaux sont traités par une combinaison logique ET (33), **en ce que** le résultat de sorte de la combinaison ET (33) est traité en association avec le premier signal dans une combinaison logique OU (35) et **en ce qu'**une alarme d'occlusion est déclenchée lors que la combinaison OU (35) est satisfaite.

Fig. 1

EP 2 052 752 B1

Fig. 2

Fig. 4

Fig. 3

Fig. 5

Fig. 6

## Kraftverläufe

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6659980 B
- US 20070191770 A

- WO 2007093064 A1

**In der Beschreibung aufgeführte Nicht-Patentliteratur**

- *IEEE Transactions on Signal Processing,* November 2001, vol. 49 (11), 2713-2730

- **R. C. Kavanagh.** *FIR Differentiators for Quantized Signals*